Europäisches Patentamt

European Patent Office

Office européen des brevets

(11) Publication number: **0 352 651**
**A1**

## EUROPEAN PATENT APPLICATION

(21) Application number: **89113414.0**

(22) Date of filing: **21.07.89**

(51) Int. Cl.⁴: **C12N 15/01 , C12N 15/11 , C07H 21/04 , C07K 13/00 , C12P 21/02**

Claims for the following Contracting States: ES + GR.

The microorganisms have been deposited with the Fermentation Research Institute under numbers FERM BP-1858 and FERM BP-1473.

(30) Priority: **26.07.88 JP 184538/88**

(43) Date of publication of application:
**31.01.90 Bulletin 90/05**

(84) Designated Contracting States:
**AT BE CH DE ES FR GB GR IT LI LU NL SE**

(71) Applicant: **HOECHST JAPAN LIMITED**
**10-16, 8-chome, Akasaka, Minato-ku**
**Tokyo(JP)**

(72) Inventor: **Hashimoto, Tamotsu**
**1-5-10, Sakae-cho**
**Asaka-shi Saitama-ken(JP)**
Inventor: **Sato, Masahiro**
**3-7-22, Minami-dai**
**Kawagoe-shi Saitama-ken(JP)**

(74) Representative: **Klein, Otto, Dr. et al**
**Hoechst AG Zentrale Patentabteilung**
**Postfach 80 03 20**
**D-6230 Frankfurt am Main 80(DE)**

(54) Human protein C mutants and method for preparing the same.

(57) A mutant protein of human protein C in which the connecting dipeptide consisting of the 156th lysine and the 157th arginine residues are either replaced by other amino acid residue(s) or deleted is disclosed.

The mutant protein C is produced by culturing host cells transfected with a plasmid vector containing DNA coding it. DNA coding for replacing amino acid residue(s) can be easily synthesized and ligated with remaining DNA portion of protein C gene.

EP 0 352 651 A1

## Human protein C mutants and method for preparing the same

Detailed Explanation of the Invention

(1) Applied fields in industry:

The present invention is related to novel single chain protein C mutants which have an anti-coagulant activity.

(2) Technological background:

Human protein C is a precursor to a serine protease called "activated protein C" present in plasma and plays an important physiological role in the regulation of blood coagulation. It is converted to an activated form by restricted proteolysis in a reaction catalyzed by thrombin or a protease from viper venom, called "protac." Activated protein C exhibits strong anti-coagulant activity which is due to its inactivation of factor VIIIa and factor Va in the presence of calcium ion (Marler, R.A., Kleiss, A.J. and Griffin, J., Blood 59, 1067-1072 (1982); Vehar, G.A. and Davie, E.W., Biochem. 19, 401-410 (1980)). The activation of protein C by thrombin is greatly accelerated by a cofactor named thrombomodulin (Esmon, C.T. and Owen, W.G., Proc. Natl. Acad. Sci. USA 78, 2249-2252 (1981)). It also accelerates the fibrinolytic system by inactivating tissue plasminogen activator inhibitor (t-PAI). DNA sequence of cDNA encoding human protein C has been determined by Foster and Davie (Proc. Natl. Acad. Sci USA 81, 4766-4770 (1984)).

Human protein C is a vitamin K-dependent glycoprotein with nine residues of $\gamma$-carboxyglutamic acid at 6, 7, 14, 16, 19, 20, 25, 26 and 29 positions from the amino- terminal. Such a $\gamma$-carboxyglutamic acid-rich region is called Gla domain and thought to play an important role in the anti-coagulant activity of protein C by interacting with the phospholipid present on the endothelial cell surface. The role of the Gla domain is described in detail in the literature "Taisha" (Metabolism), vol.19, No.9 (1982).

(3) Purpose of the invention:

It is an object of the invention to provide a novel protein C mutant by altering a portion of the amino acid sequence of human protein C. The resulting mutant protein C has properties in that the activation rate by a thrombin/thrombomudulin complex is much reduced and that the duration time of the activity is eventually extended.

(4) Construction of the invention:

The process of blood coagulation leading to the formation of a fibrin clot involves complex interactions among a variety of blood and tissue components. During the process, a series of enzymatic cascades is involved; one zymogen, a precursor protein involving blood coagulation, is converted to an activated form by another activated blood coagulating factor and the activated protein, in turn, activates the next zymogen.

At the final stage of blood coagulation, prothrombin is converted to an activated thrombin by an activated factor Xa in the presence of a cofactor, activated factor Va. The active- form thrombin converts fibrinogen to fibrin. It also activates factor VIII and factor V to accelerate the blood coagulation reaction as a positive feedback. On the other hand, thrombin functions to convert protein C to an active-form in a negative feedback mechanism. The activation of protein C by thrombin is greatly accelerated by the cofactor thrombomodulin present on the surface of endothelial cells (Esmon, C.T. and Owen, W.G., Proc. Natl. Acad. Sci. USA 78, 2249-2252 (1981)). The activated protein C serves as an anti-coagulant factor to inactivate both active-form factors Va and VIIIa (Kisiel, W. et al., Biochem. 16, 5824-5831 (1977); Marlar, R.A., Kleiss, A.J. and Griffin, J.H., Blood 59, 1067-1072 (1982)). Furthermore, the activated protein C inactivates t-PAI. As a result, formation of plasmin from plasminogen which causes degradation of fibrin clots is accelerated. Thus, protein C is thought to serve not only as an anti-coagulant factor but also as a fibrinolytic factor (Sakata Y. et al., Proc. Natl. Acad. Sci. 82, 1121-1125 (1985)). Patients with low plasma levels of protein C are apt to be attacked by thrombosis (Griffin, J.H. et al., J. Clin. Invest. 68, 1370-1373 (1981); (Griffin, J.H. et al., Blood 60, 261-264 (1982); Comp, P.C. et al., J. Clin. Invest. 74 2082-2088 (1984)-). In patients exhibiting virtual absence of protein C characterized by the homozygous state, severe fetal thrombosis is frequently observed to occur in the neonatal period (Selingsohn, U. et al., New Engl. J. Med. 310, 559-562 (1984)).

Human protein C is a vitamin K-dependent glycoprotein with an apparent molecular weight (MW) of 62-kilodalton (kd). It has nine residues of $\gamma$-carboxylglutamic acid and one equivalent of $\beta$-hydroxyaspartic acid (HO-Asp) (Kisiel, W. J., Clin. Invest. 64, 761-769 (1979)) cleaved by a signal peptidase after the alanine (Ala) residue at position -10. This yields a zymogen with a highly basic propeptide of nine amino acid residues. Processing to the mature protein that circulates in plasma involves additional proteolytic cleavage after residues at -1, 155, and 157 to remove the amino-terminal propeptide and the Lys-Arg dipeptide that connects the light and heavy chains. The two-chain form of protein C is then converted to an activated protein C by a thrombin/thrombomodulin complex by the cleavage after Arg residue at position 169. In vivo, however, the processing of the single-chain precursor seems to be incomplete because in human plasma about 5-10% of the protein C exists as a single-chain molecule (Miletich, J.P. et al., Blood 62, Suppl. 1, 306a (1983)).

Virus-free recombinant proteins have been widely thought to be a useful therapeutic reagent. The inventors have already isolated the cDNA encoding human protein C and succeeded in producing recombinant protein C in cultured mammalian cells after transfection of the cDNA whose transcription is under the regulation of various promoters. In addition, it has recently been found that the single-chain polypeptides of recombinant protein C, from which single-chain and two-chain molecules could be biochemically separated, exhibited a slower rate of activation by a thrombin/thrombomodulin complex than the two-chain polypeptides, suggesting that the former polypeptides could manifest their enzymatic activity slowly in vivo and that the duration time of the activity is possibly extended.

Human protein C mutants of this invention have the amino acid sequence which is different in positions 156 and 157 from the native sequence Lys-Arg. Namely one or both of the said two amino acids is (are) deleted or replaced by an amino acid or amino acids different from those in the native sequence, or one of the said two is deleted and the other replaced. As a result, it is expected that the human protein C mutant is characterized as a single-chain molecule of protein C. In order to create single-chain protein C, it is desirable to replace the Lys-Arg sequence by other two amino acids. Alternatively, it is also possible to replace the Lys-Arg sequence by one amino acid or three or more amino acids. Furthermore, deletion of the same dipeptide also produces a single-chain protein. As for the substitution of Lys-Arg sequence with other amino acids, it is important to utilize amino acids with as similar chemical properties to Lys and Arg as possible. In consideration of hydrophilicity, ion-charge, effectiveness to the tertiary structure of a protein and so on, some neutral amino acids including serine (Ser), asparagine (Asn) and glutamine (Gln) are preferred. The sequences including Asn-Ser, Ser-Asn, Gln-Ser, Ser-Gln, Asn-Gln or Gln-Asn are suitable for the substitution.

Human protein C mutants of this invention can be created by altering a part of DNA sequence encoding protein C by using a protein engineering technique. More precisely, such modified DNA can be prepared by replacing a part of cDNA encoding human protein C by a chemically synthesized DNA fragment. The DNA encoding protein C mutants mentioned above is expressed in a host when it is linked to an appropriate expression vector and its vector DNA is introduced into the host chromosomes. In this case, an expression vector is to be designed to function in the particular host. On the other hand, eukaryotic cells in which post-translational modifications such as glycosylation, $\gamma$-carboxylation or $\beta$-hydroxylation of a protein can be made are desirable as a host. For this purpose, cultured mammalian cells such as Chinese hamster ovary (CHO) cells and baby hamster kidney (BHK) cells are preferable.

Creation of recombinant human single-chain protein C mutants may be made by any means with the various steps including construction of mutant genes, construction of a transcription unit for expression of mutant genes in a host, introduction of expression vector containing transcription units into the host cells, selection of transformants, and biochemical characterization of recombinant proteins secreted in the culture medium.

(5) Effects of the Invention:

Recombinant single-chain human protein C mutants possess an anti-clotting activity, evaluated by thrombin/thrombomodulin- catalyzed activation system of protein C, ability to inactivate activated-form factor Va and VIIIa, and to neutralize t-PAI. It is also activated more slowly in vitro than the two-chain polypeptides of protein C, suggesting the possibility that effects of single-chain protein C are slower-activating and more durable in normal blood, i.e. possess a longer biological half-life.

(6) Explanation by examples:

The invention will be described below with Reference Examples and Examples. In the examples, the DNA coding for human protein C described in Japanese Patent Application No.96341/1987 (Laid-Open Unexamined Publication No.263083/1988) was used. It is described in Reference Example 1. The expression method described in Japanese Patent Application No.96340/1987 (Laid-Open Unexamined Publication No.267288/1988) was used. It is described in Reference Examples 2 and 3. The examples and reference examples are not intended to limit the invention.

Reference Example 1 (Construction of pCs1)

The inventors constructed a human protein C-coding gene consisting of 1389 bp of nucleotides and additionally having an EcoRI site at one end, and Smal and HindIII sites at the other end. The gene was prepared on the basis of the nucleotide sequence coding human protein C published by Foster et al., ibid., with some changes of bases without changing amino acid residues.

Plasmid pPC1 was formed by integrating the gene between EcoRI and HindIII of the plasmid vector which was derived from pUC9 (commercially available from Pharmacia) and had the same polylinker site as in pUC9 and an ampicillin-resistant gene lost with PvuI. The plasmid was transformed into E. coli K-12/Om225 which was deposited with the Fermentation Research Institute, Agency of Industrial Science and Technology as FERM BP-1858.

Then the expression vector pCs1 was constructed from the plasmid above. Namely, the plasmid pSVAstop1, in which a fragment containing the SV40 early promoter and a fragment containing a polylinker for insertion of the gene and the SV40 early mRNA polyadenylation site were connected to the 2.3 Kbp PvuII-EcoRI fragment of the plasmid pBR322 (which contains an ampicillin-resistant gene and a duplication initiation site), was cleaved at the XbaI site present in the polylinker and formed to a flush end with $T_4$ DNA polymerase. Afterwards the pPC1 was digested with EcoRI and Smal, and the 1.4 Kbp DNA fragment containing a protein C gene was isolated and purified by polyacrylamide gel electrophoresis and converted to a flush end with $T_4$ DNA polymerase, which was then ligated with $T_4$ DNA ligase to the flush end of the plasmid pSVAstop1.

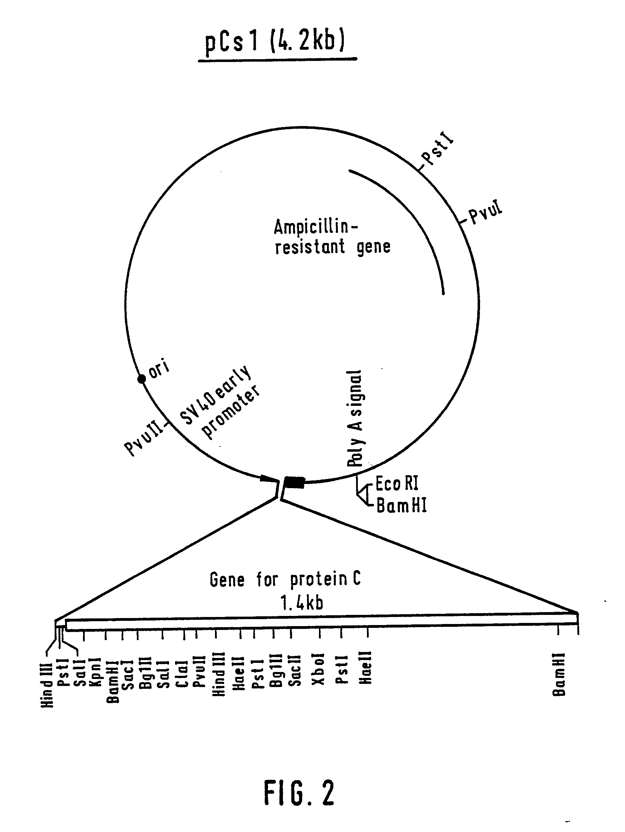

The recombinant DNA plasmids were examined to select a clone in which a protein C gene was integrated in the orientation expressible under the SV40 early promoter. The selected clone was named pCs1. The plasmid was transformed into E. coli K-12/HB101 which was deposited with the Fermentation Research Institute as FERM BP-1473. The restriction map is shown in Fig. 2.

Reference Example 2 (Construction of pCs4)

pCs4 was constructed from pCs1. pCs4 has two BstXI sites upstream from the SV40 early promoter and downstream from the poly A signal. The BstXI site was devised to produce the following asymmetric cohesive ends by digestion with BstXI.

$$5' \cdots\cdots CTGG \boxed{Gene} CCACGGGG \cdot 3'$$
$$3' \cdot CCCCGACC \boxed{} GGTG \cdots\cdots 5'$$

The fragments link with each other necessarily in tandem. This makes it possible to prepare DNA comprising many tandem repeats of an expression unit of protein C (a promoter plus a protein C gene plus a poly A signal). Protein C is produced by cultivating host animal cells transfected with the DNA comprising many tandem repeats. The production process of pCs4 is as follows: the fragment obtained by digesting pCs1 with EcoRI was ligated with the double-stranded oligonucleotide synthesized chemically as mentioned below.

4

```
5′ pAATTG [CCA] CGGGGC [TGG] C·····3′
3′ ·····C [GGT] GCCCCG [ACC] GAGCTp5′
```

wherein p represents the phosphate group coupled at the 5′ ends for the ligation, ⃞ indicates the BstXI recognition site, and indicates the cleavage site by the said enzyme.

The left side of the oligonucleotide is arranged so as to be a 5′ protruding sequence which can be ligated to the cohesive end of EcoRI-cleaved pCs1, which does not regenerate the EcoRI site. This arrangement is made so that the EcoRI site produced in the subsequent step will be a unique site. The right side is the portion that is linked with the XhoI cohesive end.

The ligation product was digested with XhoI followed again by the ligation. Both ends of the pCs1 cleaved with EcoRI were linked respectively with one molecule of the synthetic oligonucleotide. Both ends of the product were subjected to the ligation (wherein the XhoI site is formed) to form a circular plasmid.

Then, the resulting plasmid was partially digested with PvuII. As there were two PvuII sites in pCs1, the cleavage took place at either both, one, or none of the sites to give a mixture of them. Accordingly, the mixture was subjected to size fractionation by means of the electrophoresis using agarose gel to isolate the product in which the PvuII site located upstream of SV40 early promoter only had been cleaved. The isolated DNA chain was ligated with a chemically synthesized double-stranded oligonucleotide with the chemical structure shown below.

```
5′ pTT [CCA] GCCCCG [TGG] ·····3′
3′ ·AA [GGT] CGGGGC [ACC] TTAAp5′
```

wherein symbols are as defined above, and the right side is the portion that is linked with the section cleaved by EcoRI.

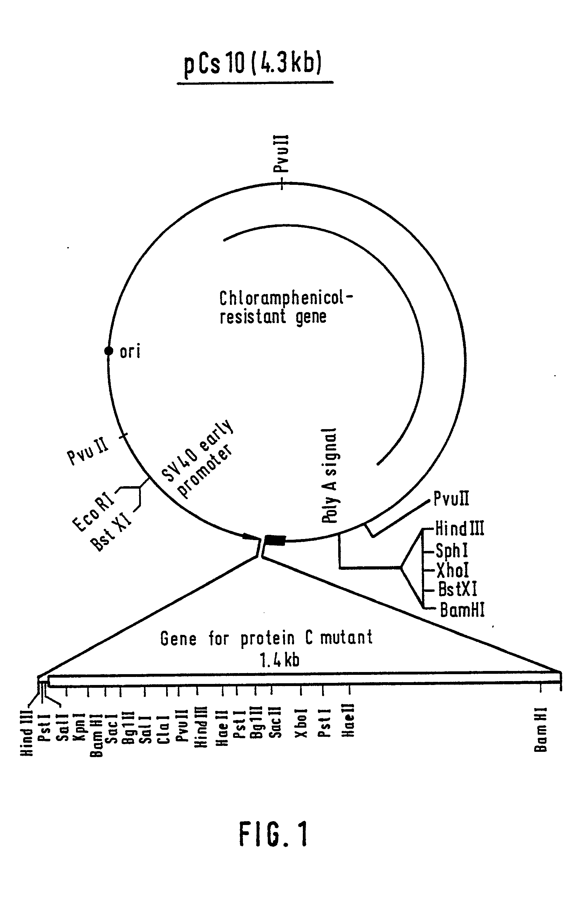

The left side of the oligonucleotide was linked with the PvuII site of the cleaved plasmid DNA, which did not regenerate the PvuII site. The ligation product was digested with EcoRI followed again by the ligation. Both ends of the PvuII-cleaved plasmid DNA were linked respectively with one molecule of the synthetic oligonucleotide. Both ends of the product were subjected to ligation (wherein the EcoRI site is located) to form a circular plasmid. The resulting plasmid was cleaved with XhoI and EcoRI, and the fragment was cloned into pHSG396 having a chloramphenicol-resistant marker (available from Takara Shuzo Co., Ltd.) cleaved with XhoI and EcoRI. The chloramphenicol-resistant protein C expression vector plasmid thus obtained was named pCs4. Its restriction map is substantially the same as that of pCs10 shown in Fig. 1.

Example 1 (Construction of pCs10)

The DNA sequence encoding the connecting dipeptide Lys-Arg of protein C is located between the BglII and SacII sites of the protein C cDNA present in both pCs1 and pCs4. In order to replace the native Lys-Arg sequence by Asn-Ser sequence, a short DNA fragment in which the 5′ and 3′ ends have a structure recognized by BglII and SacII restriction enzymes, respectively, was chemically synthesized. The DNA sequence of this fragment is shown below.

PCNS - α:GATCTCACCTGAACTCCGACACAGAAGACCAAGAAGACCAAGTAGATCCGC

PCNS- β: AGTGGACTTGAGGCTGTGTCTTCTGGTTCTTCTGGTTCATCTAGG

(wherein the underlined portions AACTCC, encoding Asn-Ser, correspond to the native connecting dipeptide Lys-Arg [AAACCA]. Other sequence remains unchanged.) Single-stranded DNAs, PCNS-α and PCNS-β , were synthesized by a DNA synthesizer (Applied Biosystem Co. and freeze-dried, 40 nmol of each oligonucleotide was then dissolved in 160 μl of distilled $H_2O$. 16 μl of each of the solutions was taken out and mixed with 2 μl of PNK buffer diluted 10-fold (500 mM Tris-Cl, pH 7.6, 100 mM MgCl$_2$, 10 mM 2-mercaptoethanol, 25 mM ATP) and 2 μl of T$_4$ polynucleotidekinase (5 units). The mixture was then reacted at 37°C for one hour. 10 μl of each reaction mixture was taken out and mixed together. The mixture was allowed to stand at 65°C for 10 min., and then annealed by placing it at room temperature over 45 min.

On the other hand, a 356-bp DNA fragment containing a Lys-Arg-coding region was isolated by digesting pCs4 DNA with ClaI and XbaI followed by size fractionation in agarose gel. This fragment was then subcloned between the ClaI and XbaI sites of pHSG396 to give rise to p396pCClxb/5. In a 356-bp ClaI-XbaI fragment, the Lys-Arg-coding sequence is present between the BglII and SacII sites of protein C cDNA. In order to replace the BglII -SacII fragment of the native protein C gene by a chemically synthesized oligonucleotide (PCNS-α/PCNS-β), p396pCCl-xb/5 was first digested with BglII and SacII restriction enzymes. After removal of the smaller BglII-SacII fragments in agarose gel electrophoresis, the isolated larger fragment containing the chloramphenicol-resistant gene was ligated with the above oligonucleotides in the presence of T$_4$ DNA ligase. The ligated mixture was then transformed into E. coli K-12/Om225. DNA sequence analysis was made for the region of the ClaI-XbaI fragment inserted in a vector using the dideoxy-chain termination method as described by Sanger et al.

As a result, it was revealed that the introduced oligonucleotide was correctly inserted and its sequence was the same as initially designed. Subsequently, a ClaI-XbaI fragment was excised from the plasmid DNA to transfer it between the ClaI and XbaI sites of pCs4, from which a small ClaI and XbaI fragment had been removed. The resulting plasmid is named pCs10. pCs10 contains a cDNA encoding a protein C mutant protein in which the Lys-Arg connecting dipeptide is replaced by Asn-Ser. The restriction map of pCs10 is shown in Fig. 1. The DNA sequence of the protein C mutant and its amino acid sequence deduced from the DNA sequence are also shown in Table 1.

Reference Example 3 (Construction pf pHSG293)

pHSG293 was also constructed which had a neo gene as a selection marker and BstXI site providing the same asymmetric cohesive ends as pCs10 by digestion. The fragment obtained by digesting pHSG293 with BstXI was ligated with the fragment obtained by digesting pCs10 so that the selection of transfectants was possible. A production process is as follows: when pHSG274 deposited as ATCC 37301, a cosmid vector with the neo gene which confers kanamycin resistance to E. coli host and G418 (geneticin) resistance in eucaryotic cells was digested with BstXI, cleavage took place as shown in the following flow sheet. Then a synthetic nucleotide shown in the flow sheet was ligated.

pHSG274
↓ Bst XI

```
   ┌───┐                        ┌───┐
   │CCA│ TCATG            A     │TGG│ ---- HindⅢ ────┐
   │GGT│ A          GTACT       │ACC│                │
   └───┘                        └───┘                │
 cos └──────────────────────┐                        │
                            │                        │
Synthetic                 ┌───┐         ┌───┐        │
Oligonucleotide  GTAC     │CCA│ GCCCCG  │TGG│         A
                          │GGT│ CGGGGC  │ACC│-XbaI-lacOP-  │
                          └───┘         └───┘        TTCGA
   (BstXI⁻)                  BstXI                (HindⅢ)
```

↓ Ligation

```
                  ┌───┐         ┌───┐
  ─ BstXI⁻ ─      │CCA│ GCCCCG  │TGG│            A
                  │GGT│ CGGGGC  │ACC│-XbaI-lacOP-
                  └───┘         └───┘          TTCGA
      ┌──────────     BstXI                 (HindⅢ)
 cos  │
      │                                  ┌───┐
      │                          A       │TGG│ ----- HindⅢ ──┐
      │                    GTACT         │ACC│               │
      └──────────────────────────────── └───┘ ──────────────┘
```

↓ HindⅢ

```
                  ┌───┐         ┌───┐
  ─ BstXI⁻ ─      │CCA│ GCCCCG  │TGG│            A
                  │GGT│ CGGGGC  │ACC│-XbaI-lacOP-
                  └───┘         └───┘          TTCGA
      ┌──────────     BstXI                 (HindⅢ)
 cos  │
      │                                       (HindⅢ)┐
      └───────────────────────────────────────────────┘
```

↓ Ligation

pHSG 293

↓ BstXI

**3.6kb fragment**

The left side of the synthetic oligonucleotide was linked with the cosmid vector DNA cleaved by BstXI and the BstXI site was destroyed. Subsequent digestion of the cleavage product with HindIII results in removal of the HindIII-BstXI portion contained in the cosmid vector and the excessive synthetic repeatedly ligated fragments. The resulting product was subjected to ligation to afford a circular plasmid in which the right side of the synthetic oligonucleotide was linked with the HindIII site of the cosmid vector.

The Xbal site in the synthetic oligonucleotide sequence was introduced to distinguish pHSG274 and pHSG293 from each other. The lac operator in the synthetic oligonucleotide was introduced to select transformants by kanamycin resistance after cosmid-packaging and to distinguish transformants that form blue colonies in the presence of X-gal.

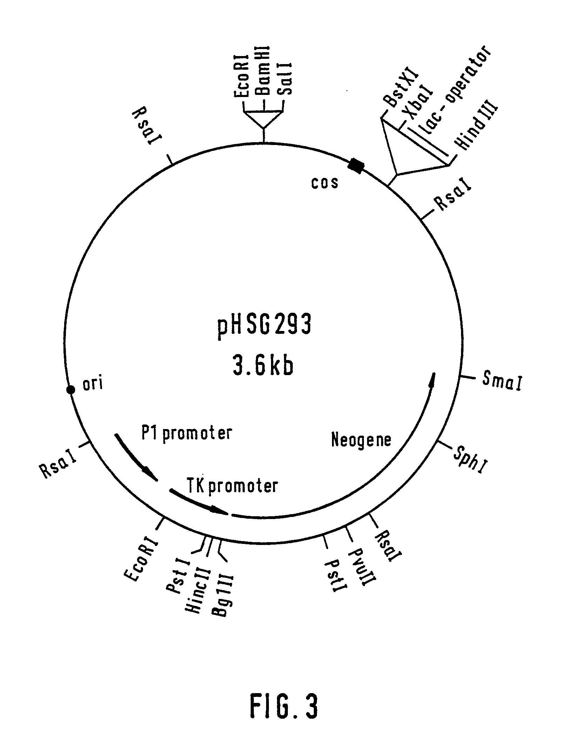

The plasmid was named pHSG293. Its restriction map is shown in Fig. 3.

Example 2

In vitro amplification of DNA fragments containing single-chain mutant protein C-expressing units and its large-scale preparation in E. coli.

When the pCs10 DNA described in Example 1 is digested with BstXI, a 2.2-Kb fragment containing transcription unit and a 2.1-Kb fragment of the vector portion are obtained. However, it is difficult to isolate each fragment in a purified state by size-fractionation after electrophoresis in agarose gel. To overcome this problem, pCs10 DNA was digested with EcoRI restriction enzyme and the 4.3-Kb linearized fragment was inserted into the EcoRI site of pHSG11. pHSG11, 9.1-Kb in size, is a derivative of a temperature-sensitive variant of pSC101, pHSG1 (Hashimoto-Gotoh, T. and Sekiguchi, M.J., Bacteriology 31, 405-412 (1977)), from which the single BstXI site has been destroyed by DNA polymerase. The resulting plasmid, named pCs11, has 13.4-Kb in size and contains a 11.2-Kb vector component containing the chloramphenicol-resistant gene derived from pCs10 and the tetracycline-resistant gene from pHSG11, and a 2.2-Kb transcription unit. The pCs11 DNA was digested with BstXI and electrophoresed in 0.7% agarose gel to purify the 2.2-Kb fragment. On the other hand, pHSG293 DNA was digested with BstXI and subsequently treated with calf intestine alkaline phosphatase (CIAP). 2.0 $\mu$g of 2.2-Kb BstXI fragment containing a protein C mutant expression unit was mixed with 0.1 $\mu$g of BstXI-digested and CIAP-treated pHSG293 DNA at a ratio of 10:1 in 10 $\mu$l of solution containing $T_4$ DNA ligase (5 units).

Five microliters of ligation mixture was taken out and then packaged into $\lambda$phage particles by using an in vitro packaging kit (Stratagene Co.). The reaction was carried out according to the manufacturer's protocol. DH-1 cells, one of E. coli K-12 strains, were then infected with the phage particles and allowed to grow overnight at 37°C on LS agar plates containing 25 $\mu$g/ml of kanamycin. Each colony formed was transferred to 100 ml of a liquid LS medium containing kanamycin and incubated overnight at 37°C. From this culture, about 10 $\mu$g of cosmid DNA is recovered through a standard procedure of plasmid DNA isolation. Circular cosmid DNA with ca. 45-Kb in size was digested with BstXI to yield two kinds of DNA fragments, an 11.2-Kb fragment containing pHSG293 vector and a 2.2-Kb fragment containing transcription units. After electrophoresis of BstXI-digested cosmid DNA on 0.7% agarose gel containing 0.5 $\mu$g/ml of etidium bromide (EtBr), the intensity of each band that had been photographed under UV-illumination was measured by densitometric scanning. As a result, a typical cosmid carrying 8 copies of transcription units and 2 copies of pHSG293 vectors was obtained. This cosmid was named pCNS701.

Example 3

Production of a single-chain protein C mutant in cultured animal cells and demonstration of protein C-like activity

pCNS701 cosmid DNA was introduced into BHK and CHO-dhfr⁻ (lack of dehydroforate reductase activity) cells by a standard transfection method described by Wigler et al. $1 \times 10^6$ cells were seeded on a tissue culture dish (100 mm in diameter, Falcon, 3001) in 8 ml of Dulbecco's modified Eagle's medium (DME) supplemented with 10% heat-inactivated fetal bovine serum (FBS) and 40 $\mu$g/ml of proline in the case of BHK or of nucleoside-containing Minimum Essential Eagle's medium (MEM-$\alpha$) supplemented with 10% FDS in the case of CHO-dhfr⁻. These cells were cultured for about 20 hours at 37°C in an atmosphere of 5% $CO_2$/95% air by the time when the cell growth reaches at 20% confluency. For transfection, 7 $\mu$g of pCNS701 was dissolved in 900 $\mu$l of 140 mM NaCl, 0.75 mM NaHPO₄ and 25 mM HEPES (pH 7.1), and finally 60 $\mu$l of 2 M $CaCl_2$ was added to the solution. The mixture was gently mixed and then allowed to stand for at least 30 min. at room temperature. The resulting mixture in a volume of 960 $\mu$l was dropwise added to the medium in the above-mentioned culture dishes. The mixture was incubated for 24 hours at 37°C to allow the exogenous DNA to be incorporated into the cells. Then, the culture

8

medium was replaced by 8 ml of a fresh medium, and cultivation was made for additional 18 hours. Fourty-two hours after transfection, cells were collected by a trypsin/EDTA treatment and split to 10 dishes (100 mm in diameter). Each dish, which contains 8 ml of culture medium supplemented with 10% FBS, 400 $\mu$g/ml of G-418 (GIBCO) and 40 $\mu$g/ml of proline (in the case of BHK), was incubated for 7-14 days at 37°C. 85 BHK-derived and 57 CHO-dhfr⁻-derived G-418-resistant colonies per $\mu$g of pCNS701 were obtained. From each colony, single cell clones were isolated and then propagated.

For biochemical analyses including ELISA (enzyme-linked immunoabsorbent assay) and anti-clotting assay, $1 \times 10^6$ cells were seeded in a tissue culture dish (60 mm in diameter, Falcon, 3002) in which 3 ml of culture medium and 0.1 $\mu$g/ml of vitamin K were contained, and incubated for 24 hours at 37°C. After one day in culture, the medium was replaced by a fresh medium (3 ml in volume) containing 0.1 $\mu$g/ml of vitamin K, and cultivation was made for additional 24 hours. The culture medium was then collected, divided into aliquots (1 ml in volume) and stored at -70°C up to the biochemical analysis.

To determine the content of the human protein C mutant secreted in the culture medium, an ELISA was carried out. Fifty-six microliters of affinity-purified sheep polyclonal antibodies to human protein C (5 $\mu$g protein/ml) was placed in each well of a 96-well microtiter plate (Petra), and the plate was then allowed to stand for one hour at room temperature.

Then, the antibody was removed and 250 $\mu$l of PBS (phosphate-buffered saline, pH 7.2) containing 1% bovine serum albumin (BSA) was added to each well. After the plate was allowed to stand for 30 min. at room temperature, the solution in each well was removed. After washing four times with 200 $\mu$l of PBS, the plate was air-dried and stored at 4°C until use. 40 $\mu$l of frozen-thawed culture medium was poured onto each antibody-coated well of a microtiter plate, and then the plate was left for one hour at room temperature. Then, the solution was removed and the well was washed four times with 200 $\mu$l of PBS. Subsequently, 40 $\mu$l of sheet anti-human protein C antibody labeled with horse radish peroxidase (HRP) was added into each well, and the plate was left for one hour at room temperature. After removal of the antibody and subsequent washing with PBS, 50 $\mu$l of citrate-buffered solution containing 1 mg/ml of orthophenyldiamine (OPD) and 0.006% of $H_2O_2$ was poured into each well, followed by standing for 10-15 min. at room temperature. Then, the reaction product exhibiting reddish brown color was quantified by an ELISA plate reader (Dynatech). About 47% of the G-418-resistant clones secreted 0.5 $\mu$g/ml or more of protein C mutant in the culture medium as shown in Table 2. Particulary, the B-7 clone originated from BHK cells and the C-5 clone originated from CHO-dhfr⁻ cells exhibited higher levels of production of 0.74 $\mu$g/ml and 0.94 $\mu$g.ml, respectively.

Next, in order to examine whether protein C mutants secreted have a biological activity, an anti-coagulant assay was carried out by using an assay kit provided by Behringwerke (Cat. No. 2576, 2577, OTXA 11). This assay is based upon the ability of protein C activated by "protac" to inhibit (or delay) blood coagulation in vitro. As shown in Table 2, B-7 and C-5 exhibited anti-coagulant activity in the levels of 2.7 and 11.0% of that of control normal plasma, respectively.

Example 4

Demonstration that single-chain molecules of protein C are predominantly secreted from the cells carrying DNA encoding the human protein C mutant in their chromosomes.

In order to determine whether the recombinant protein C mutant secreted in the medium consists of predominantly single-chain molecules or not, the culture medium was analyzed by Western blotting hybridization using polyclonal antibody to human protein C.

$1 \times 10^6$ cells of B-7 or C-5 were independently seeded on a tissue culture dish (60 mm in diameter, Falcon, 3002) and cultured in the same medium mentioned above for 3 days. The culture dish was then washed twice with serum-free culture medium and cultivated in the same medium for additional 24 hours. After culture, 1 ml of supernatant was removed, mixed with 333 $\mu$l of 100% trichloroacetic acid (TCA) in an Eppendorf tube (1.5 ml volume), and the mixture was left for 30 min. at 0°C.

After centrifugation at 15,000 rpm for 15 min., the precipitates were dissolved in 20 $\mu$l of sample buffer, and then ca. 1 $\mu$l of saturated Trisma base solution was added to the solution to adjust the pH to 7.0-7.5. The suspension usually contains 0.5-1 $\mu$g of protein. The samples were then separated on SDS-PAGE and electrophoretically transferred to a nylon filter in the presence of 25 mM Tris-192 mM glycine (pH 8.3)-20% ethanol. Subsequently, the filter was fixed in 20 mM Tris-HCl (pH 7.5)-500 mM NaCl for 60 min. at room temperature. Then, the filter was subjected to an immunostaining; it was reacted with goat anti-human protein C antibody, washed twice, incubated with rabbit anti-goat IgG antibody labeled with HRP, and finally washed twice.

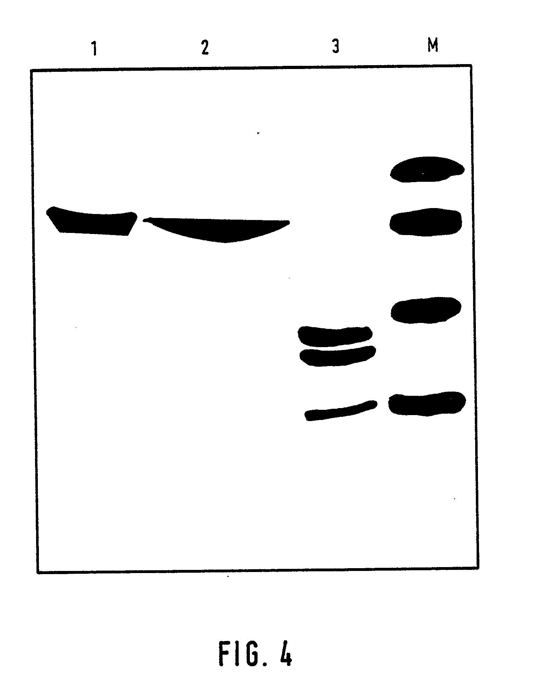

As shown in Fig. 4, the protein C purified from human plasma exhibits two major bands, a heavy-chain molecules with a MW of about 40-Kd (in Fig. 4 isoforms of a heavy-chain polypeptide, $\alpha$- and $\beta$-chains, are observed) and a light-chain molecules of 21-Kd in size. Another minor band with a MW of 62-Kd, which corresponds to a single-chain form of protein C, is also found.

On samples from B-7 and C-15, only a major band with a MW of 62-Kd can be observed (see Fig. 4, lanes 1 and 2).

These results indicate that unlike human protein C of plasma origin whose connecting dipeptide Lys-Arg is processed by a signal peptidase, the recombinant human protein C mutant is resistant to an enzymatic attack by the same protease, possibly due to the presence of the protease-insensitive dipeptide Asn-Ser.

EP 0 352 651 A1

## Table 1

```
                                                                      -42
                                                                      ATG. TGG.
                                                                      Met-Trp-

-40                                              -30
CAG. CTC. ACA. AGC. CTC. CTG. CTG. TTC. GTG. GCC. ACC. TGG. GGA. ATT. TCC. GGT. ACC. CCA. GCT. CCT.
Gln-Leu-Thr-Ser-Leu-Leu-Leu-Phe-Val-Ala-Thr-Trp-Gly-Ile-Ser-Gly-Thr-Pro-Ala-Pro-

-20                                              -10
CTT. GAC. TCA. GTG. TTC. TCC. AGC. AGC. GAG. CGT. GCC. CAC. CAG. GTG. CTG. CGG. ATC. CGC. AAA. CGT.
Leu-Asp-Ser-Val-Phe-Ser-Ser-Ser-Glu-Arg-Ala-His-Gln-Val-Leu-Arg-Ile-Arg-Lys-Arg-

1                                                10                                            20
GCC. AAC. TCC. TTC. CTG. GAG. GAG. CTC. CGT. CAC. AGC. AGC. CTG. GAG. CGG. GAG. TGC. ATA. GAG. GAG.
Ala-Asn-Ser-Phe-Leu-Glu-Glu-Leu-Arg-His-Ser-Ser-Leu-Glu-Arg-Glu-Cys-Ile-Glu-Glu-

                                                 30                                            40
ATC. TGT. GAC. TTC. GAG. GAG. GCC. AAG. GAA. ATT. TTC. CAA. AAT. GTG. GAT. GAC. ACA. CTG. GCC. TTC.
Ile-Cys-Asp-Phe-Glu-Glu-Ala-Lys-Glu-Ile-Phe-Gln-Asn-Val-Asp-Asp-Thr-Leu-Ala-Phe-

                                                 50                                            60
TGG. TCC. AAG. CAC. GTC. GAC. GGT. GAC. CAG. TGC. TTG. GTC. TTG. CCC. TTG. GAG. CAC. CCG. TGC. GCC.
Trp-Ser-Lys-His-Val-Asp-Gly-Asp-Gln-Cys-Leu-Val-Leu-Pro-Leu-Glu-His-Pro-Cys-Ala-

                                                 70                                            80
AGC. CTG. TGC. TGC. GGG. CAC. GGC. ACG. TGC. ATC. GAT. GGC. ATC. GGC. AGC. TTC. AGC. TGC. GAC. TGC.
Ser-Leu-Cys-Cys-Gly-His-Gly-Thr-Cys-Ile-Asp-Gly-Ile-Gly-Ser-Phe-Ser-Cys-Asp-Cys-
```

EP 0 352 651 A1

90                                                                                        100
CGC. AGC. GGC. TGG. GAG. GGC. CGC. TTC. TGC. CAG. CGC. GAG. GTA. AGC. TTC. CTC. AAT. TGC. TCT. CTG.
Arg-Ser-Gly-Trp-Glu-Gly-Arg-Phe-Cys-Gln-Arg-Glu-Val-Ser-Phe-Leu-Asn-Cys-Ser-Leu-

110                                                                                       120
GAC. AAC. GGC. GGC. TGC. ACG. CAT. TAC. TGC. CTA. GAG. GAG. GTG. GGC. TGG. CGG. CGC. TGT. AGC. TGT.
Asp-Asn-Gly-Gly-Cys-Thr-His-Tyr-Cys-Leu-Glu-Glu-Val-Gly-Trp-Arg-Arg-Cys-Ser-Cys-

130                                                                                       140
GCG. CCT. GGC. TAC. AAG. CTG. GGG. GAC. GAC. CTC. CTG. CAG. TGT. CAC. CCC. GCA. GTG. AAG. TTC. CCT.
Ala-Pro-Gly-Tyr-Lys-Leu-Gly-Asp-Asp-Leu-Leu-Gln-Cys-His-Pro-Ala-Val-Lys-Phe-Pro-

150                                                                                       160
TGT. GGG. AGG. CCC. TGG. AAG. CGG. ATG. GAG. AAG. AAG. AGA. TCT. CAC. CTG. AAC. TCC. GAC. ACA. GAA.
Cys-Gly-Arg-Pro-Trp-Lys-Arg-Met-Glu-Lys-Lys-Arg-Ser-His-Leu-Asn-Ser-Asp-Thr-Glu-

170                                                                                       180
GAC. CAA. GAA. GAC. CAA. GTA. GAT. CCG. CGG. CTC. ATT. GAT. GGG. AAG. ATG. ACC. AGG. CGG. GGA. GAC.
Asp-Gln-Glu-Asp-Gln-Val-Asp-Pro-Arg-Leu-Ile-Asp-Gly-Lys-Met-Thr-Arg-Arg-Gly-Asp-

190                                                                                       200
AGC. CCC. TGG. CAG. GTG. GTC. CTT. CTA. GAC. TCA. AAG. AAG. AAG. CTG. GCC. TGC. GGG. GCA. GTG. CTC.
Ser-Pro-Trp-Gln-Val-Val-Leu-Leu-Asp-Ser-Lys-Lys-Lys-Leu-Ala-Cys-Gly-Ala-Val-Leu-

210                                                                                       220
ATC. CAC. CCC. TCC. TGG. GTG. CTG. ACT. GCA. GCC. CAC. TGC. ATG. GAT. GAG. TCC. AAG. AAG. CTC. CTT.
Ile-His-Pro-Ser-Trp-Val-Leu-Thr-Ala-Ala-His-Cys-Met-Asp-Glu-Ser-Lys-Lys-Leu-Leu-

EP 0 352 651 A1

230                                                        240
GTC. AGG. CTT. GGA. GAG. TAT. GAC. CTG. CGG. CGC. TGG. GAG. AAG. TGG. GAG. CTG. GAC. CTG. GAC. ATC.
Val-Arg-Leu-Gly-Glu-Tyr-Asp-Leu-Arg-Arg-Trp-Glu-Lys-Trp-Glu-Leu-Asp-Leu-Asp-Ile-

250                                                        260
AAG. GAG. GTC. TTC. GTC. CAC. CCC. AAC. TAC. AGC. AAG. AGC. ACC. ACC. GAC. AAT. GAC. ATC. GCA. CTG.
Lys-Glu-Val-Phe-Val-His-Pro-Asn-Tyr-Ser-Lys-Ser-Thr-Thr-Asp-Asn-Asp-Ile-Ala-Leu-

270                                                        280
CTG. CAC. CTG. GCC. CAG. CCC. GCC. ACC. CTC. TCG. CAG. ACC. ATA. GTG. CCC. ATC. TGC. CTC. CCG. GAC.
Leu-His-Leu-Ala-Gln-Pro-Ala-Thr-Leu-Ser-Gln-Thr-Ile-Val-Pro-Ile-Cys-Leu-Pro-Asp-

290                                                        300
AGC. GGC. CTT. GCA. GAG. CGC. GAG. CTC. AAT. CAG. GCC. GGC. CAG. GAG. ACC. CTC. GTG. ACG. GGC. TGG.
Ser-Gly-Leu-Ala-Glu-Arg-Glu-Leu-Asn-Gln-Ala-Gly-Gln-Glu-Thr-Leu-Val-Thr-Gly-Trp-

310                                                        320
GGC. TAC. CAC. AGC. AGC. CGA. GAG. AAG. GAG. GCC. AAG. AGA. AAC. CGC. ACC. TTC. GTC. CTC. AAC. TTC.
Gly-Tyr-His-Ser-Ser-Arg-Glu-Lys-Glu-Ala-Lys-Arg-Asn-Arg-Thr-Phe-Val-Leu-Asn-Phe-

330                                                        340
ATC. AAG. ATT. CCC. GTG. GTC. CCG. CAC. AAT. GAG. TGC. AGC. GAG. GTC. ATG. AGC. AAC. ATG. GTG. TCT.
Ile-Lys-Ile-Pro-Val-Val-Pro-His-Asn-Glu-Cys-Ser-Glu-Val-Met-Ser-Asn-Met-Val-Ser-

350                                                        360
GAG. AAC. ATG. CTG. TGT. GCG. GGC. ATC. CTC. GGG. GAC. CGG. CAG. GAT. GCC. TGC. GAG. GGC. GAC. AGT.
Glu-Asn-Met-Leu-Cys-Ala-Gly-Ile-Leu-Gly-Asp-Arg-Gln-Asp-Ala-Cys-Glu-Gly-Asp-Ser-

```
                           370                                                                380
GGG. GGG. CCC. ATG. GTC. GCC. TCC. TTC. CAC. GGC. ACC. TGG. TTC. CTG. GTG. GGC. CTG. GTG. AGC. TGG.
Gly-Gly-Pro-Met-Val-Ala-Ser-Phe-His-Gly-Thr-Trp-Phe-Leu-Val-Gly-Leu-Val-Ser-Trp-

                           390                                                                400
GGT. GAG. GGC. TGT. GGG. CTC. CTT. CAC. AAC. TAC. GGC. GTT. TAC. ACC. AAA. GTC. AGC. CGC. TAC. CTC.
Gly-Glu-Gly-Cys-Gly-Leu-Leu-His-Asn-Tyr-Gly-Val-Tyr-Thr-Lys-Val-Ser-Arg-Tyr-Leu-

                           410                                                                420
GAC. TGG. ATC. CAT. GGG. CAC. ATC. AGA. GAC. AAG. GAA. GCC. CCC. CAG. AAG. AGC. TGG. GCA. CCT. TAG.
Asp-Trp-Ile-His-Gly-His-Ile-Arg-Asp-Lys-Glu-Ala-Pro-Gln-Lys-Ser-Trp-Ala-Pro-Ter-

TAA
Ter
```

wherein Ter is the termination codon.

EP 0 352 651 A1

Table 2

| Clone | ELISA ($\mu$g/ml) | Anti-blood coagulatory activity (%)* |
|---|---|---|
| BHK-origin cells | | |
| B-1 | 0.39 | ND** |
| B-2 | 0.395 | ND |
| B-3 | 0.52 | ND |
| B-4 | 0.47 | ND |
| B-6 | 0.555 | ND |
| B-7 | 0.74 | 2.7 |
| B-8 | 0.405 | ND |
| B-9 | 0.535 | 4.0 |
| B-10 | 0.555 | ND |
| B-11 | 0.375 | ND |
| B-12 | 0.65 | 11.0 |
| CHO-origin cells | | |
| C-3 | 0.12 | ND |
| C-4 | 0.15 | 6.0 |
| C-5 | 0.94 | 11.0 |
| C-11 | 0.335 | ND |
| C-15 | 0.285 | 4.5 |
| C-22 | 0.065 | ND |

* Anti-blood coagulatory activity is expressed as the percent of that of protein C contained in human plasma used as a control.
** Not determined.

Brief Description of the Drawings:

Fig. 1 is a restriction map of pCs10.
Fig. 2 is a restriction map of pCs1.
Fig. 3 is a restriction map of pHSG293.
Fig. 4 is a protein profile when analyzed by Western blotting of the human protein C mutant secreted in the culture medium by transformants.
Lane 1: Supernatant from the culture of B-7 transformant
Lane 2: Supernatant from the culture of C-5 transformant
Lane 3: Protein C purified from normal human plasma
Lane M: Protein molecular weight marker (from the top, bands for 97 kd, 68 kd, 43 kd, 26 kd and 18 kd)

## Claims

1. A human protein C mutant wherein the amino acid sequence is different in positions 156 and 157 from the native sequence Lys-Arg.

2. A mutant as claimed in claim 1, wherein one or both of the said two amino acids is (are) deleted or replaced by an amino acid or amino acids different from those in the native sequence or wherein one of the said two amino acids is deleted and the other is replaced by an amino acid different from that in the native sequence.

3. A mutant as claimed in claim 1 or 2, wherein one or both of the said two amino acids is (are replaced by an amino acid selected from the group consisting of Ser, Asn and Gln.

4. A mutant as claimed in claim 3, wherein the said two amino acids are replaced by Asn-Ser.

5. A DNA coding for a human protein C mutant wherein the amino acid sequence is different in positions 156 and 157 from the native sequence Lys-Arg.

6. A DNA coding for a mutant as claimed in claim 5, wherein one or both of the said two amino acids is (are) deleted or replaced by an amino acid or amino acids different from those in the native sequence or wherein one of the said two amino acids is deleted and the other is replaced by an amino acid different from that in the native sequence.

7. A DNA coding for a mutant as claimed in claim 5 or 6, wherein one or both of the said two amino acids is (are) replaced by an amino acid selected from the group consisting of Ser, An and Gln.

A DNA coding for a mutant as claimed in claim 7, wherein the said two amino acids are replaced by Asn-Ser.

9. A process for preparing a human protein C mutant as defined in one or more of claims 1 to 4, wherein a DNA as defined in one or more of claims 5 to 8 is caused to be expressed in a suitable eukaryotic host cell.

10. A process as claimed in claim 9, wherein the host cell is an animal cell, preferably a mammalian cell.

Claims for the following Contracting States: ES,GR

1. A process for the preparation of a human protein C mutant the amino acid sequence of which is different in positions 156 and 157 from the native sequence Lys-Arg, which comprises expressing in a suitable eukaryotic host cell a DNA coding for the said human protein C mutant.

2. A process as claimed in claim 1, wherein the DNA is coding for a mutant, in which one or both of the said two amino acids is (are) deleted.

3. A process as claimed in claim 1, wherein the DNA is coding for a mutant, in which one or both of the said two amino acids is (are) replaced by an amino acid or amino acids different from those in the native sequence.

4. A process as claimed in claim 1, wherein the DNA is coding for a mutant, in which one of the said two amino acids is deleted and the other is replaced by an amino acid different from that in the native sequence.

5. A process as claimed in claim 3, wherein the DNA is coding for a mutant, in which one or both of the said two amino acids is (are) replaced by an amino acid selected from the group consisting of Ser, Asn and

16

Gln.

6. A process as claimed in claim 5, wherein the DNA is coding for a mutant, in which the said two amino acids are replaced by Asn-Ser.

7. A process as claimed in one or more of the preceding claims, wherein the DNA is expressed in an animal cell.

8. A process as claimed in claim 7, wherein the cell is a mammalian cell.

pCs 10 (4.3kb)

FIG. 1

pCs1 (4.2kb)

FIG. 2

FIG.3

FIG. 4

## DOCUMENTS CONSIDERED TO BE RELEVANT ·

EP 89113414.0

| Category | Citation of document with indication, where appropriate, of relevant passages | Relevant to claim | CLASSIFICATION OF THE APPLICATION (Int Cl.X) 5 |
|---|---|---|---|
| X | EP - A2 - 0 266 190 (ZYMOGENETICS INC.) * Claim 6 * | 1,5 | C 12 N 15/01 C 12 N 15/11 C 07 H 21/04 C 07 K 13/00 C 12 P 21/02 |
| P,A | EP - A2 - 0 296 413 (HOECHST JAPAN LIMITED) * Abstract * | 1,5,9, 10 | |
| A | EP - A1 - 0 215 548 (ZYMOGENETICS, INC.) * Claims; fig. 2 * | 1,5,9, 10 | |
| D,A | PROCEEDINGS OF THE NATIONAL ACADEMY OF SCIENCES OF THE UNITED STATES OF AMERICA, vol. 81, no. 15, August 1984 (Baltimore, USA) D. FOSTER et al. "Characterization of a cDNA coding for human protein C" pages 4766-4770 * Totality * | 1,5 | |

TECHNICAL FIELDS SEARCHED (Int. Cl.X) 5

C 12 N
C 07 H
C 07 K
C 12 P

The present search report has been drawn up for all claims

| Place of search VIENNA | Date of completion of the search 24-10-1989 | Examiner WOLF |
|---|---|---|